# EUROPEAN PATENT SPECIFICATION

(11) **EP 0 474 841 B1**
(45) Date of publication and mention of the grant of the patent: **15.06.1994**
(21) Application number: 91907321.3
(22) Date of filing: 04.04.1991
(51) Int. Cl.: A61F 2/64

(54) **ARTIFICIAL LIMB COMPONENT**
BESTANDTEIL EINES KÜNSTLICHEN GLIEDES
CONSTITUANT DE MEMBRE ARTIFICIEL

(30) Priority: 06.04.1990 GB 9007829
(43) Date of publication of application: 18.03.1992
(73) Proprietor: CHAS. A. BLATCHFORD & SONS LIMITED, Basingstoke Hampshire RG22 4AH (GB)
(72) Inventor: WOOLNOUGH, Victor James, North Waltham, Hampshire (GB); EARLY, Stanley Thomas, Basingstoke, Hampshire RG22 5HE (GB)
(74) Representative: Blatchford, William Michael
(86) International application number: GB9100528
(87) International publication number: WO9115169

(56) References cited:
- EP-A- 0 226 278
- EP-A- 0 350 337
- FR-A- 1 526 399
- GB-A- 2 162 069
- US-A- 4 379 350

## Description

### ARTIFICIAL LIMB COMPONENT

This invention relates to a turntable for an artificial limb, which is arranged to allow rotation of an upper component of the limb relative to a lower component of the limb about a longitudinal axis, as specified in the preamble of claim 1. Such a turntable is disclosed, for example, in EP-A-0226278.

It is well known to provide a lower limb prosthesis for an above-knee amputee with a turntable located proximally of the knee. One of the primary functions of the turntable is to allow the amputee to sit cross-legged. Conventionally, the turntable has a lock for preventing rotation during normal use of the limb but which is generally manually releasable so that the amputee may, when required, rotate the lower part of the limb including the knee about the longitudinal axis.

In the past, it has proved difficult to design a turntable which is effective in operation, reliable, and yet relatively inexpensive to manufacture.

The present invention provides a turntable for connecting together an upper component and a lower component of an artificial limb such that the two components are rotatable with respect to each other about a longitudinal turntable rotation axis, characterised in that the turntable comprises: an inner member having a head portion and a neck portion for connecting the head portion to one of the said limb components, and an outer member for connection to or associated with the other of the said limb components and having a radially inwardly extending portion adjacent the neck portion of the inner member whereby the head portion is rotatably trapped by the outer member, and in that the turntable further comprises annular adjustable bearing means encircling the axis, the bearing means being associated with the inner member and bearing against a generally radially extending surface of the outer member so as to urge a bearing surface of the inner member head portion against a bearing surface of the outer member. By appropriate adjustment of the bearing means, thereby altering the thrust applied to the outer member, play between the bearing surfaces can be largely eliminated. Play, which may be caused by manufacturing tolerances or by wear, can seriously affect the effectiveness of the limb.

In the preferred embodiment of the invention the bearing means comprises a collet of adjustable size arranged to act against the outer member and an opposing surface associated with the inner member. Either the collet itself or one of the surfaces it engages, or both, may be inclined when viewed in a transverse cross-section so that if the collet is adjusted in diameter the outer member is forced away from the opposing surface to urge the inter-engaging bearing surfaces of the inner and outer members together. In a particularly advantageous arrangement the collet is circular and has an inwardly tapering cross-section.

Preferably, the collet, when in use, is secured so as to rotate with the inner member, and has an inwardly directed conical surface which bears against a corresponding outwardly directed conical surface on the outer member. Since the inner member is rotatable with respect to the outer member and, therefore, the collet is also rotatable with respect to the outer member, it is possible, by tightening the collet beyond the point at which play is eliminated, to produce a required degree of resistance to rotation in use of the turntable due to friction between the inter-engaging conical surfaces.

The collet is preferably manufactured from a flexible thermoplastics material such as a polyacetal, which is sufficiently flexible to allow adjustment of diameter, and is in the form of a ring with a single generally radial gap bridged by a tangentially oriented adjusting screw.

In the preferred embodiment of the invention, the head and neck portions of the inner member are integrally formed with an axial threaded stud for securing the inner member to the top plate of a knee mechanism. The collet may be located on the outer circumferential edge of a washer fitted on the stud and positioned to be trapped between the inner member neck portion and the knee mechanism top plate when the inner member is secured to a top plate. By providing an inwardly directed groove on the inner edge of the collet and a similar outwardly directed groove on the outer edge of the washer it is possible to locate the collet on the washer by means of an O-ring located in the grooves. It has been found that the presence of a rubber O-ring of a suitable size between the collet and the washer not only serves to locate the collet on the washer but prevents it rotating with respect to the washer when the outer member rotates. The resistance to rotation offered by the O-ring is aided in the preferred embodiment by cut-outs in the inner edge of the collet, into which the O-ring is free to expand. The resilience of the O-ring assists in releasing the collet bearing surfaces should stiction occur during adjustment, and it has the advantage of keeping the collet located on the washer during assembly of the turntable to the knee mechanism. Side play of the collet with respect to the washer is also avoided.

The outer member preferably has a concave spherical socket-mounting surface remote from the inner member for receiving the corresponding convex spherical end surface of a stump socket which may be secured to the outer member by an axial bolt passing through a plate inside the socket, and through a hole in the end of socket to engage a central threaded bore in the outer member.

The invention also includes a turntable comprising two members which are rotatable with respect to each other about a longitudinal axis of the limb and which may be locked together to prevent such rotation by means of a locking device, wherein the locking device is mounted in one of the members and has a locking pawl which is inwardly tapered in a transverse cross-section and is generally radially movable into and out of a correspondingly tapered recess in the other member. By arranging for the locking device to be spring loaded, so that the tapered pawl is urged into the tapered recess, rotational play between the members in the locked position is largely eliminated. The angle of the taper is sufficiently large that the possibility of the pawl becoming jammed in the recess due to friction between the inter engaging surfaces of the pawl recess is largely avoided.

The invention includes a lower limb prosthesis incorporating a turntable as described above.

The invention will now be described by way of example with reference to the drawings in which:-
Figure 1 is a vertical cross-section of part of a lower limb prosthesis for an above-knee amputee and including a turntable in accordance with the invention, the prosthesis being viewed from one side;
Figure 2 is a plan view of the turntable of Figure 1, the sectioning line for Figure 1 being shown by the line I - I;
Figure 3 is a top plan view of an adjustable collet and washer assembly forming part of the turntable of Figures 1 and 2;
Figure 4 is a detail cross-section through the collet;
Figure 5 is an underside view of an upper part of the turntable showing a locking device; and
Figure 6 is a transverse cross-section of the upper part shown in Figure 5 along with the line VI - VI in Figure 5.

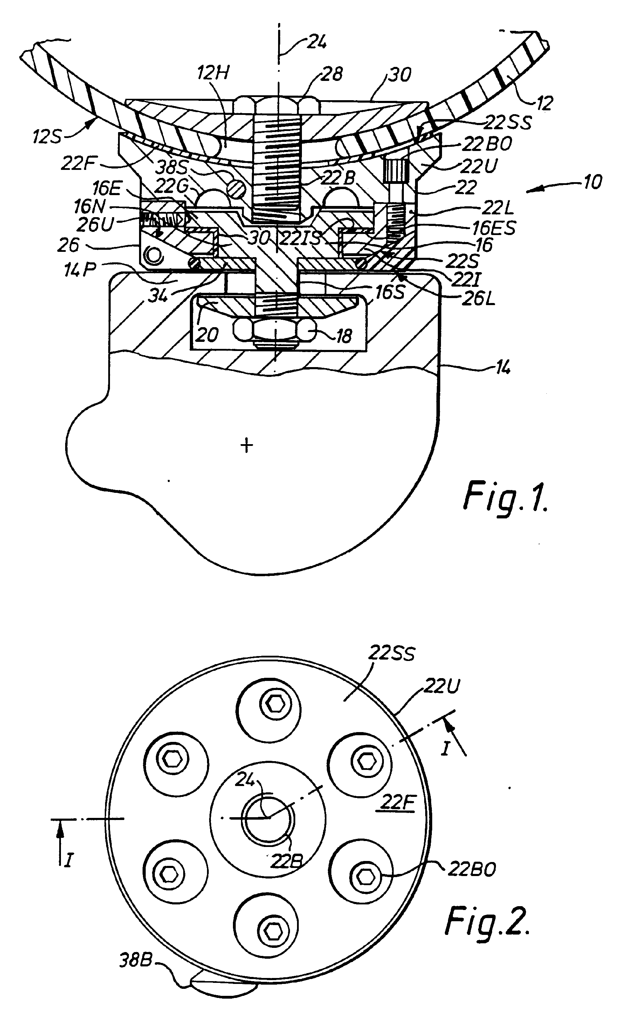

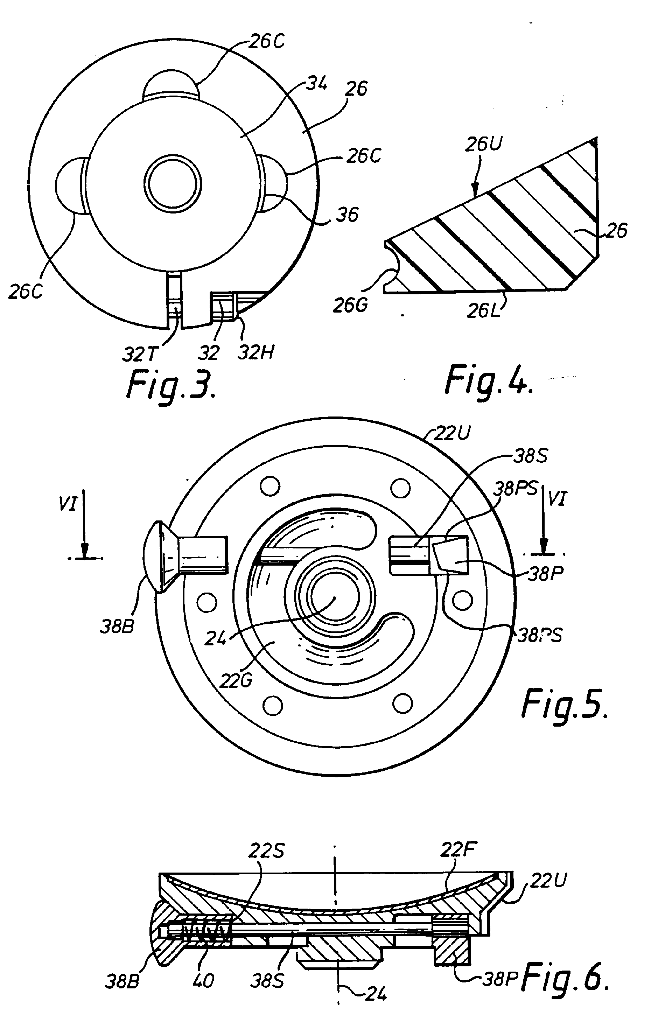

Referring to Figures 1 and 2, a turntable 10 in accordance with the invention serves to connect a plastics stump socket 12 (preferably formed by polypropylene) and a knee mechanism 14 of a lower limb prosthesis for an above-knee amputee. The turntable has three main parts, which are an inner member 16 with a depending stud 16S for attaching the turntable to the top plate 14P of the knee mechanism 14 by means of a nut 18 threaded on the stud 16S and a washer 20 beneath the plate, an outer member 22 surrounding the inner member 16 and allowing the latter to rotate inside it about a longitudinally axis 24 of the limb, and an adjustable collet 26 of inwardly tapering cross-section located between an outwardly directed lower conical surface 22S of the outer member 22 and the top plate 14P of the knee mechanism.

The inner member has a head portion 16E, and a neck portion 16N which is of smaller diameter than the end portion 16E and which connects the latter to the stud 16S. By virtue of its larger diameter, the end portion 16E defines an annular groove around the neck portion 16N and below a radially extending bearing surface 16ES of the end portion 16E. The outer member 22 surrounds the neck and end portions of the inner member 16, and is formed in two parts as follows. An upper part 22U has a concave part-spherical seating surface 22SS for receiving a corresponding convex part-spherical lower surface 12S of the stump socket 12. The socket 12 is attached to the upper part 22U of the outer member 22 by means of a bolt 28 which passes through a plate 30 internal to the socket, and through a hole 12H at the end of the socket into a threaded bore 22B in the upper part 22U. A layer of friction material 22F is bonded to the concave surface 22SS to prevent movement of the socket 12 on the surface when the bolt 28 has been tightened. The spherical shape of the concave surface 22SS and of the corresponding convex surface 12S of the socket 12, in conjunction with the relatively large hole in the polypropylene material of the socket allows a degree of alignment adjustment to be formed by moving the turntable relative to the socket. The upper part 22U of the outer member 22 overlies the end portion 16E of the inner member and is bolted by means of bolts 22BO to a lower part 22L of the outer member having a portion 22I which extends inwardly to encircle the neck portion 16N of the inner member 16. This inwardly extending portion 22I has an upper radially extending bearing surface 22IS facing the bearing surface 16ES on the inner member end portion 16E. Since the inwardly extending portion 22I of the outer member lies within the groove defined by the end and neck portions 16E and 16N of the inner member 16, the inner member 16 is trapped within the outer member 22 and is rotatable relative to the outer member about the longitudinal axis 24. The inner member 16 has a nylon or similar plastics bearing lining 30 of top-hat cross-section which bears against the outer member 22, to reduce friction.

As described so far, the inner member 16, although trapped within and rotatable within the outer member 22, is free to move axially by a small amount. This axial play between the members 16 and 22 is taken up by interposing the collet 26 between the lower conical surface 22S of the outer member 22 and the top plate 14P of the knee mechanism 14. The collet 26 is of inwardly tapering cross-section, having a lower surface 26L which engages the knee mechanism top plate 14P, and an inwardly and upwardly directed concave conical surface 26U which engages the corresponding convex conical surface 22S of the outer member 22.

Referring to Figures 3 and 4, the collet 26 comprises a ring of the ring so formed being bridged by a screw 32 having a head 32H seated in one end portion of the ring and a threaded portion 32T threaded in a bore in the other end portion of the ring. The diameter of the collet 26 is thus adjustable, and it will be appreciated that the conical cross section of the collet, as shown in the detail of Figure 4, will result in the outer member 22 of the turntable being urged upwardly against the end portion 16E of the inner member 16 when the screw 32 is tightened. Consequently, it is possible to eliminate the vertical play between the inner and outer members 16 and 22.

In this embodiment of the invention, the collet 26 is located on the outer edge of a washer 34 encircling the stud 16S of the inner member 16. This washer is trapped between the inner member 16 and the top plate 14P of the knee mechanism, and is prevented from rotating with respect of the knee mechanism when the nut 18 is tightened. In its outer edge, the washer 34 has a groove of semi-circular cross-section for receiving a rubber O-ring 36. A similar groove 26G is cut in the inner edge of the collet 26 for receiving the outer part of the O-ring 36. It follows, that the collet 26 is flexibly mounted on the washer 34, and can therefore be adjusted in diameter. Yet the O-ring 36 grips both collet 26 and washer 34 effectively to prevent rotation of the collet 26 with respect to the knee mechanism 14. As a result, the conical upper surface 26U of the collet 26 will slide over the corresponding surface 22S of the outer member 22 when the inner member 16 is rotated within the outer member 22. It has been mentioned above that tightening of the collet adjusting screw 32 can be used to eliminate vertical play between the inner and outer members 16 and 22 of the turntable. Further tightening of the screw 32 increases the friction between the collet 26 and the outer member 22, which can be beneficial in some circumstances.

The collet 26 is preferably made of a plastics material such as polyacetal so as to be both light and sufficiently flexible to be adjusted in diameter. Three cut-outs 26C increase the flexibility of the collet 26 and have the secondary function of increasing the resistance to rotation of the collet 26 about the washer 34 since the O-ring 36 expands into these cut-outs.

Further advantages of the flexible O-ring mounting of the collet 26 include the ability to keep the collet 26 and washer 34 together during assembly of the turntable 10 to the knee mechanism 14, and the biasing of the collet in a direction tending to increase its diameter so as to assist in releasing the opposing conical surfaces of the collet 26 and outer member 22 should stiction develop during adjustment.

The use of a collet 26 with a conical surface also substantially prevents rocking of the outer member 22 with respect to the inner member 16, and the O-ring 36 resists side play.

During normal use of the prosthesis, the turntable is locked by a locking device. When, however, the user wishes to sit cross-legged, or encounters difficulty in, for example, putting on a shoe, the locking device is released so as to allow the knee mechanism 14 and the lower part of the limb to be rotated with respect to the stump socket 12. One form of locking device will now be described with reference to Figures 5 and 6.

As stated above with reference to Figure 1, the outer member 22 of the turntable is split into an upper part 22U and a lower part 22L. When the upper part 22U is viewed from the underside, the locking device is visible, as shown in Figure 5. Still referring to Figure 5, but now in conjunction with the sectional view of Figure 6 which is a section along the line VI - VI in Figure 5, the locking device comprises a locking pawl 38P mounted on one end of a locking shaft 38S which extends transversely in the upper part 22U of the outer member offset to one side of the longitudinally axis 24. At the other end of the shaft 38S there is mounted a locking button 38B accessible to the user. As will be seen from the section of Figure 6, a compression spring 40 is mounted on the shaft 38S between the locking button 38B and a shoulder 22US on the outer member upper part 22U, thereby biasing the locking pawl 38P inwardly away from the outer periphery of the outer member 22. In Figure 6, the spring 40 is shown compressed, with locking pawl 38P in its outer position, allowing rotation of the turntable. Still referring to Figure 6, the locking pawl 38P projects downwardly from the outer member upper part 22U to engage in a notch (not shown) in the inner member 16 of the turntable when the pawl and notch are in registry with each other and the button 38B is released. The pawl 38P has side surfaces 38PS at least one of which is angled so that the pawl is inwardly tapered in the direction of the locking button 38B. The notch in the inner member 16 is similarly tapered. This has the effect of positively preventing rotational play between the inner and outer members 16 and 22 when the lock is engaged. However, the angle of inclination of the side surfaces of the pawl 38P is arranged such that the possibility of the pawl 38P being forced out of the notch in the inner member 16 due to a rotational force being applied to the turntable is avoided. Conversely, the angle of inclination is sufficiently great to avoid the pawl 38P becoming jammed in the notch. When the pawl 38P is moved out of the notch, and the turntable rotated, release of the button 38B results in the pawl 38P sliding over an outer cylindrical surface of the inner member 16 (not shown) as rotation continues.

## Claims

1. A turntable (10) for connecting together an upper component (12) and a lower component (14) of an artificial limb such that the two components are rotatable with respect to each other about a longitudinal turntable rotation axis (24), characterised in that the turntable comprises: an inner member (16) having a head portion (16E) and a neck portion (16N) for connecting the head portion (16E) to one of the said limb components, and an outer member (22) for connection to or associated with the other of the said limb components and having a radially inwardly extending portion (22I) adjacent the neck portion of the inner member whereby the head portion (16E) is rotatably trapped by the outer member, and in that the turntable further comprises annular adjustable bearing means (26) encircling the axis (24), the bearing means (26) being associated with the inner member (16) and bearing against a generally radially extending surface (22S) of the outer member (22) so as to urge a bearing surface (16ES) of the inner member head portion (16E) against a bearing surface (22IS) of the outer member (22).

2. A turntable according to claim 1, characterised in that the bearing means comprises a collet (26) of adjustable size arranged to act against the outer member (22) and an opposing surface associated with the inner member.

3. A turntable according to claim 2, characterised in that either the collet (26) itself, or one of the surfaces it engages, or both, are inclined when viewed in a transverse cross-section whereby adjustment of the collet size causes the outer member (22) to be forced away from the said opposing surface to urge the inter-engaging bearing surfaces (16ES, 22IS) of the inner and outer members together.

4. A turntable according to claim 2 or claim 3, characterised in that the collet (26) is circular and has an inwardly tapering cross-section.

5. A turntable according to any of claims 2 to 4, characterised in that the collet comprises a ring (26) of flexible thermoplastics material and with a generally radial break bridged by a tangentially oriented adjusting screw (32).

6. A turntable according to claim 5, characterised in that the ring (26) has an inner edge with an inwardly directed annular groove (26G), the ring (26) being mounted on an elastomeric O-ring (36) received in the said groove and in an outwardly directed groove in the inner member (16).

7. A turntable according to claim 6, characterised in that the outwardly directed groove is formed in the outer edge of a washer (34) forming part of the inner member (16).

8. A turntable according to claim 6 or claim 7, characterised in that the inner edge of the ring (26) is provided with cut-outs (26C) into which the O-ring (36) can expand.

9. A turntable according to any preceding claim, characterised in that the annular adjustable bearing means (26) encircle the neck portion (16N) of the inner member (16).

10. A turntable according to claim 1, characterised in that the annular adjustable bearing means comprises a flexible collet in the form of a ring (26) having an inwardly tapered cross-section and a break which is spanned by a tangential adjusting screw (32) for adjusting the diameter of the ring, in that the outer member (22) has a mounting surface (22SS) for mounting the said other limb component, and an annular surface (22S) which, in an axial cross-section of the outer member (22), is inclined so as to face both in the opposite direction from the mounting surface (22SS) and outwardly away from the axis (24), the annular surface (22S) engaging a corresponding inclined surface (26U) on the annular bearing means (26), and in that the inner member (16) is so formed that, in use, the annular bearing means (26) is sandwiched between the inclined surface (22S) of the outer member (22) and an opposing surface of the inner member or the said one limb component, whereby tightening of the tangential screw (32) causes the outer member (22) to be forced away from the said opposing surface so as to urge the bearing surface (16ES) of the inner member head portion (16E) against the said bearing surface (22IS) of the outer member (22), thereby to eliminate play between the inner and outer members.

11. A turntable according to claim 1, characterised in that the inner member neck portion (16N) has an integral threaded stud (16S) for engaging in a threaded hole in the said one limb component.

12. A turntable according to any preceding claim, characterised by a locking device mounted in one of the inner and outer members (16, 22) and having a locking pawl (38P) which is inwardly tapered in a transverse cross-section and is generally radially movable into and out of a correspondingly tapered recess in the other member.

13. A lower limb prosthesis including a turntable according to any preceding claim.

## Patentansprüche

1. Drehscheibe (10), die einen oberen Bestandteil (12) und einen unteren Bestandteil (14) eines künstlichen Gliedes so miteinander verbindet, daß die zwei Bestandteile zueinander um eine in Längsrichtung verlaufende Drehachse (24) der Drehscheibe drehbar snd, **dadurch gekennzeichnet, daß** die Drehscheibe folgendes umfaßt: ein inneres Element (16) mit einem Kopfabschnitt (16E) und einem Halsabschnitt (16N), der den Kopfabschnitt (16E) mit einem der Bestandteile des Gliedes verbindet, und ein äußeres Element (22) zur Verbindung mit oder zum Anschluß an den anderen der Bestandteile des Gliedes, das außerdem einen sich radial nach innen erstreckenden Abschnitt (22I) im Bereich des Halsabschnittes des inneren Elements aufweist, so daß der Kopfabschnitt (16E) drehbar in dem äußeren Element gehalten ist, und daß die Drehscheibe des weiteren eine die Achse (24) umgebende ringförmige verstellbare Lagereinrichtung (26) umfaßt, wobei die Lagereinrichtung (26) mit dem inneren Element (16) verbunden ist und an einer im allgemeinen radial verlaufenden Fläche (22S) des äußeren Elementes (22) anliegt, um eine Lagerfläche (16ES) des Kopfabschnittes (16E) des inneren Elementes gegen eine Lagerfläche (22IS) des äußeren Elementes (22) zu drücken.

2. Drehscheibe nach Anspruch 1, **dadurch gekennzeichnet, daß** die Lagereinrichtung eine Klemmhülse (26) von verstellbarer Größe umfaßt, die so angeordnet ist, daß sie gegen das äußere Element (22) und eine zu dem inneren Element gehörige gegenüberliegende Fläche drückt.

3. Drehscheibe nach Anspruch 2, **dadurch gekennzeichnet**, **daß** die Klemmhülse (26) selbst oder eine der Flächen, mit denen sie in Eingriff steht, oder beide bei Betrachtung im Querschnitt geneigt sind, so daß die Veränderung der Größe der Klemmhülse dazu führt, daß das äußere Element (22) von der geg nüberliegenden Fläche weggedrückt wird und die ineinandergreifenden Lagerflächen (16ES, 22IS) des inneren und des äußeren Elementes zusammendrückt.

4. Drehscheibe nach Anspruch 2 oder Anspruch 3, **dadurch gekennzeichnet, daß** die Klemmhülse (26) kreisrund ist und einen sich nach innen verjüngenden Querschnitt besitzt.

5. Drehscheibe nach einem der Ansprüche 2 bis 4, **dadurch** **gekennzeichnet, daß** die Klemmhülse einen Ring (26) aus einem flexiblen thermoplastischen Material und mit einer im allgemeinen radialen Unterbrechung umfaßt, die durch eine tangential ausgerichtete Stellschraube (32) überbrückt wird.

6. Drehscheibe nach Anspruch 5, **dadurch gekennzeichnet, daß** der Ring (26) einen inneren Rand mit einer nach innen gerichteten ringförmigen Nut (26G) besitzt, wobei der Ring (26) auf einem elastomeren O-Ring (36) angeordnet ist, der in der Nut und in einer nach außen gerichteten Nut in dem inneren Element (16) aufgenommen ist.

7. Drehscheibe nach Anspruch 6, **dadurch gekennzeichnet, daß** die nach außen gerichtete Nut im äußeren Rand einer Unterlegscheibe (34) ausgebildet ist, die Teil des inneren Elementes (16) ist.

8. Drehscheibe nach Anspruch 6 oder Anspruch 7, **dadurch** **gekennzeichnet, daß** der innere Rand des Ringes (26) mit Ausschnitten (26C) versehen ist, in die sich der O-Ring (36) ausdehnen kann.

9. Drehscheibe nach einem der vorhergehenden Ansprüche, **dadurch gekennzeichnet, daß** die ringförmige verstellbare Lagereinrichtung (26) den Halsabschnitt (16N) des inneren Elementes (16) umgibt.

10. Drehscheibe nach Anspruch 1, **dadurch gekennzeichnet**, **daß** die ringförmige verstellbare Lagereinrichtung eine flexible Klemmhülse in Form eines Ringes (26) umfaßt, der einen sich nach innen verjüngenden Querschnitt und eine Unterbrechung aufweist, die durch eine tangentiale Stellschraube (32) überbrückt wird, die den Durchmesser des Ringes verändert, daß das äußere Element (22) eine Befestigungsfläche (22SS) zur Befestigung des anderen Bestandteils des Gliedes aufweist sowie eine ringförmige Fläche (22S), die im axialen Querschnitt des äußeren Elementes (22) so geneigt ist, daß sie von der Befestigungsfläche (22SS) in die entgegengesetzte Richtung und von der Achse (24) nach außen weist, wobei die ringförmige Fläche (22S) in eine entsprechende geneigte Fläche (26U) auf der ringförmigen Lagereinrichtung (26) eingreift, und daß das innere Element (16) so ausgebildet ist, daß im Gebrauch die ringförmige Lagereinrichtung (26) sandwichartig zwischen der geneigten Fläche (22S) des äußeren Elementes (22) und einer gegenüberliegenden Fläche des inneren Elementes oder dem einen Bestandteil des Gliedes gelagert ist, so daß durch das Anziehen der tangentialen Schraube (32) das äußere Element (22) von der gegenüberliegenden Fläche weggedrückt wird, um die Lagerfläche (16ES) des Kopfabschnittes (16E) des inneren Elementes gegen die Lagerfläche (22IS) des äußeren Elementes (22) zu drücken, um auf diese Weise das Spiel zwischen dem inneren und dem äußeren Element auszuschalten.

11. Drehscheibe nach Anspruch 1, **dadurch gekennzeichnet**, **daß** der Halsabschnitt (16N) des inneren Elements einen einstückigen Gewindebolzen (16S) aufweist, der in eine Gewindebohrung in dem einen Bestandteil des Gliedes eingreift.

12. Drehscheibe nach einem der vorhergehenden Ansprüche, **dadurch gekennzeichnet, daß** eine Sperrvorrichtung in einem von dem inneren und dem äußeren Element (16, 22) angeordnet ist und eine Sperrklinke (38P) aufweist, die im Querschnitt nach innen verjüngt ist und im allgemeinen in radialer Richtung in und aus einer entsprechend verjüngten Ausnehmung in dem anderen Element bewegbar ist.

13. Unterschenkelprothese umfassend eine Drehscheibe nach einem der vorhergehenden Ansprüche.

## Revendications

1. Coupelle de rotation (10) pour connecter ensemble un composant supérieur (12) et un composant inférieur (14) d'un membre artificiel de façon à ce que les deux composants soient rotatifs l'un par rapport à l'autre au tour de l'axe longitudinal (24) de rotation de ladite coupelle de rotation, caractérisée par les constituants suivants:- un élément intérieur (16) ayant une partie en forme de tête (16E) et une partie en forme de col (16N) pour connecter la partie en forme de tête (16E) dudit élément intérieur (16) à l'un desdits composants de membre artificiel, et un élément extérieur (22) qui se connecte à l'autre composant dudit membre artificiel ou s'associe avec cedit autre composant, et comportant une partie (22I) qui s'étend radialement vers l'intérieur et qui est adjacente à la partie en forme de col de l'élément intérieur (16), ce qui a pour effet de permettre à la partie en forme de tête (16E) de l'élément intérieur (16) de tourner tout en restant prisonnière de l'élément extérieur (22) , et caractérisée par le fait qu'elle comprend en outre un moyen à roulement annulaire réglable (26) encerclant l'axe 24, ledit moyen de roulement (26) étant associé à l'élément intérieur (16) et portant contre une face qui s'étend radialement (22S) de l'élément extérieur (22) pour appliquer une face de portée (16ES) de la partie en forme de tête (16E) de l'élément intérieur (16) contre une face de portée (22IS) de l'élément extérieur (22).

2. Coupelle de rotation selon la revendication 1, dans laquelle le moyen à roulement comprend un collet (26) de dimension réglable aménagé pour qu'il agisse contre l'élément extérieur (22) et contre une face opposée (22S) associée audit élément interne.

3. Coupelle de rotation selon la revendication 2, dans laquelle le profil du collet lui-même vu en coupe ou le profil d'une des faces vues en coupe sur lesquelles il s'engage, ou le profil vu en coupe à la fois du collet et de ladite surface, est incliné, ce qui permet de régler la dimension du collet pour éloigner l'élément extérieur (22) de la face opposée pour forcer les faces intérieures (16ES, 22IS) de l'élément intérieur (16) et de l'élément extérieur (22) à s'appliquer l'une contre l'autre.

4. Coupelle de rotation selon la revendication 2 ou selon la revendication 3, dans laquelle le collet (26) est circulaire et possède un profil à conicité dirigée vers l'intérieur.

5. Coupelle de rotation selon l'une quelconque des revendications 2 à 4, caractérisée par le fait que le collet comporte une couronne (26) en matériau thermoplastique flexible fendue radialement pour former un intervalle traversé perpendiculairement par une vis de réglage (32) à orientation tangentielle.

6. Coupelle de rotation selon la revendication 5, caractérisée par le fait que la couronne (26) comporte un bord intérieur ayant une gorge annulaire orientée vers l'intérieur (26G), la couronne (26) étant montée sur un joint torique (36) en élastomère logé moitié dans ladite gorge et moitié dans une gorge de l'élément intérieur (16) orientée vers l'extérieur.

7. Coupelle de rotation selon la revendication 6, caractérisée par le fait que la gorge dirigée vers l'extérieur est formée dans le bord extérieur de la rondelle (34) formant une partie de l'élément intérieur (16).

8. Coupelle de rotation selon la revendication 6 ou selon la revendication 7, caractérisée par le fait que le bord intérieur de la couronne (36) comporte des encoches (26C) dans lesquelles le joint torique (36) peut déborder.

9. Coupelle de rotation selon l'une quelconque des revendications ci-dessus, caractérisée par le fait que le moyen à roulement annulaire réglable (26) encercle la partie en forme de col (16N) de l'élément intérieur (16).

10. Coupelle de rotation selon la revendication 1, caractérisée par le fait que le moyen à roulement annulaire réglable comprend un collet flexible en forme de couronne (26) ayant un profil en pente dirigé vers l'intérieur et une fente traversée transversalement par une vis de réglage tangentielle (32) pour régler le diamètre de la couronne, et caractérisée par le fait que l'élément extérieur (22) comporte une face de portée (22SS) pour supporter ledit autre composant de membre artificiel, et une face annulaire (22S) qui sur la coupe transversale axiale de l'élément extérieur 22 a un profil incliné qui lui permet -- dans la direction opposée à la face de portée (22SS) et vers l'extérieur à partir de l'axe (24) -- de faire face à la face annulaire (22S) qui s'engage sur une face inclinée correspondante (26U) sur le moyen à roulement annulaire (26), et caractérisée par le fait que l'élément intérieur (16) est formé de manière à ce que, pendant l'usage, le moyen à roulement annulaire (26) soit maintenu entre la face inclinée (22S) de l'élément extérieur (22) et une face opposée de l'élément intérieur ou dudit composant de membre artificiel, ce qui permet de cette façon par le serrage de la vis tangentielle (32) de forcer l'élément extérieur (22) à s'éloigner de ladite face opposé pour forcer la face de portée (16ES) de la partie en forme de tête de l'élément intérieur (16E) à s'appliquer contre ladite face de portée (22IS) de l'élément extérieur et de l'élément intérieur.

11. Coupelle de rotation selon la revendication 1, caractérisée par le fait que la partie en forme de col de l'élément intérieur (16N) comporte un axe fileté (16S) qui fait partie intégrante dudit élément intérieur et qui se visse dans un trou taraudé dans ledit élément de membre.

12. Coupelle de rotation selon l'une quelconque des revendications ci-dessus, caractérisée par un dispositif de blocage monté dans l'élément intérieur (16) ou dans l'élément extérieur (22) et comportant une cliquet de verrouillage (38P) à face inclinée vers l'intérieur et pouvant se déplacer radialement de manière générale vers l'intérieur et vers l'extérieur d'un évidement ayant une face inclinée correspondante et logé dans l'autre l'élément.

13. Une prothèse de membre inférieur comprenant une coupelle de rotation selon l'une quelconque des revendications précédentes.
